# EUROPÄISCHE PATENTSCHRIFT

(11) **EP 1 280 885 B1**
(45) Veröffentlichungstag und Bekanntmachung des Hinweises auf die Patenterteilung: **19.10.2005**
(21) Anmeldenummer: 01927844.9
(22) Anmeldetag: 30.03.2001
(51) Int. Cl.: C12M 1/26, C12M 3/00

(54) **VERFAHREN ZUR ABTRENNUNG VON LEBENSFÄHIGEN SÄUGER- ODER INSEKTEN-ZELLEN AUS ZELLSUSPENSIONEN**
METHOD FOR SEPARATING VIABLE MAMMAL OR INSECT CELLS FROM CELL SUSPENSIONS
PROCEDE POUR SEPARER DES CELLULES VIABLES D'INSECTES OU DE MAMMIFERES DE SUSPENSIONS CELLULAIRES

(30) Priorität: 11.05.2000 DE 10022635
(43) Veröffentlichungstag der Anmeldung: 05.02.2003
(73) Patentinhaber: Gesellschaft für Biotechnologische Forschung mbH (GBF), D-38124 Braunschweig (DE)
(72) Erfinder: DECKWER, Wolf-Dieter, 38124 Braunschweig (DE); ANSPACH, Birger, 38124 Braunschweig (DE); DE ANDRADE MEDRONHO, Ricardo, 38124 Braunschweig (DE); LÜBBERSTEDT, Marc, 38124 Braunschweig (DE)
(74) Vertreter: Vossius & Partner
(86) Internationale Anmeldenummer: PCT/EP2001/003665
(87) Internationale Veröffentlichungsnummer: WO 2001/085902

(56) Entgegenhaltungen:
- EP-A- 0 068 537
- WO-A-92/01779
- WO-A-98/01394
- US-A- 3 647 633
- US-A- 5 547 858
- DATABASE WPI Section Ch, Week 199209 Derwent Publications Ltd., London, GB; Class D16, AN 1992-070901 XP002176099 -& SU 1 637 882 A (MOSCOW CHEM EQUIP INST) , 30. März 1991 (1991-03-30)

## Beschreibung

Die Erfindung betrifft ein Verfahren zur Abtrennung von lebensfähigen Saüger- oder Insekten-Zellen aus Zellsuspensionen unter Verwendung von Hydrozyklonen.

In den vergangenen Jahrzehnten hat die Zellkulturtechnik deutliche Fortschritte gemacht und hat speziell dazu beigetragen, hochwertige Produkte mit therapeutischem Nutzen, wie Pharmaproteine der verschiedensten Art, herzustellen. Neue Impulse gehen derzeit von der Gewebekultivierung sowie dem direkten Einsatz von Zellen und Genen für die Therapie aus. Hier wird ein hoher kommerzieller Nutzen erwartet (Peshwa, 1999). Bei der Aufarbeitung von Zellkulturen hat sich jedoch gezeigt, daß eine einfache Methode zur Abtrennung bzw. Anreicherung von lebensfähigen Zellen nicht zur Verfügung steht.

Viele Bioreaktoren, die in der Industrie zur Erzeugung biopharmazeutischer Produkte eingesetzt werden, arbeiten in der sog. Perfusionsfahrweise. Dabei wird zellfreies Kulturmedium kontinuierlich aus dem Reaktor abgezogen, während gleichzeitig Frischmedium nachgefüttert wird. Durch diese Betriebsweise erreicht man hohe Zellkonzentrationen und daher auch hohe volumetrische Produktivitäten für die gewünschten Pharmaprodukte im Vergleich zu satz- bzw. chargenweisem Betrieb bzw. Zufütterungskulturen (Batch und Fedbatch) (Zeng & Deckwer, 1999).

Für die Aberntung einer Säugerzellkultur, in welcher Betriebsart sie auch immer gefahren wurde, ist der erste notwendige Schritt die Abtrennung der Zellen aus dem Medium. Die konventionellen Methoden, um dieses Ziel zu erreichen, sind Zentrifugation, Mikrofiltration bzw. Absetzung im Erdschwerefeld.

Ein Nachteil des Einsatzes von Zentrifugen sind ihre hohen Anschaffungskosten, die hohen Betriebskosten und ihre relativ geringe Kapazität. Deshalb führt der Einsatz von Zentrifugen bei der Perfusionskultur von Säugerzellen zu erhöhten Investment- und Betriebskosten, was sich schließlich im Endpreis des Produktes widerspiegelt. Einen weiteren Nachteil von Zentrifugen stellt die Schwierigkeit ihrer Sterilisation und eines aseptischen bzw. monoseptischen Betriebs dar.

Dies gilt besonders für Tellerzentrifugen, die am häufigsten zur Zellseparation genutzt werden. Neben der In-situ-Sterilisation stellt die Wärmeerzeugung beim Betrieb von Tellerzentrifugen ein weiteres Problem dar.

Diese Probleme lassen sich jedoch erfolgreich lösen, wie ein Patent von Zentritech (Yhland, 1992) zeigt. Hierbei werden Plastikbeutel benutzt, die in den Rotor der Zentrifuge eingelegt werden und die an das Fermentationssystem ohne rotierende Abdichtungen eingelegt werden können. Die Auslegung und der Betrieb dieser Zentritech-Anlage ist jedoch recht aufwendig, von vergleichsweise geringer Kapazität und mit hohen Kosten verbunden.

Was die Mikrofiltration betrifft, so liegt das Hauptproblem hierin in der Belegung bzw. als Folge davon in der Verstopfung der Membran während des Betriebs, bei dem kontinuierlich Filtrat abgezogen wird. Das bedeutet, daß dieser Zellseparationsprozeß über einen längeren Zeitraum nicht stabil betrieben werden kann, was aber bei Perfusionssäugerzellkulturen, falls diese zur Produktion von Pharmaproteinen eingesetzt werden, erforderlich ist, und zwar über mehrere Monate hinweg.

Um das Problem der Verstopfung der Membran zu vermeiden, wurden Rotationsfilter vorgeschlagen. Eine Übersicht über diese Apparate findet man bei Tokashiki und Yokoyama (1997). Diese Autoren betonen jedoch auch, daß eine Verschmutzung bzw. Verstopfung der Membran sich kaum vollständig vermeiden läßt.

Ein weiteres wichtiges Problem beim Einsatz von Filtrationstrenntechniken mittels Membranen ist deren geringe mechanische Stabilität, die besonders bei hohen Transmembrandrücken zu deren Bruch führen kann. In diesem Fall muß der ganze Prozeß abgestellt und mindestens die Membran ausgetauscht werden, wenn nicht sogar der ganze Prozeß wegen mikrobieller Kontamination abgebrochen werden muß.

Das dynamische Filter, das von der GBF - Gesellschaft für Biotechnologische Forschung mbH vorgeschlagen und für diese patentiert wurde, besitzt einen konischen Rotor, der die Zellablagerung an der Membranoberfläche zurückdrängt, aber auch hier lassen sich langfristig eine Verstopfung und das Problem des Membranbruchs nicht vermeiden (Kroner & Vogel 1998).

Bei der Sedimentation von Säugerzellen im Erdschwerefeld beobachtet man auch bei relativ großen Zellen nur sehr geringe Absetzgeschwindigkeiten. Diese liegen nämlich nur im Bereich von 2 - 10 cm/h, so daß die Zellabtrennbarkeit hier nur sehr gering ist (Tokashiki & Yokoyama, 1997). Wegen der geringen Absetzgeschwindigkeit der Zellen muß angesichts der geringen Absetzfläche in den üblicherweise verwendeten Reaktoren die Perfusionsrate niedrig gehalten werden. Hohe Perfusionsraten lassen sich nur in Reaktoren mit extrem großen Absetzflächen realisieren. Das verursacht große Schwierigkeiten beim Scaleup dieser Reaktoren als auch bei ihrer Sterilisation. Die Absetzung im Erdschwerefeld ist deshalb von geringer Bedeutung für Perfusionssysteme.

Vor kurzem wurden neue Methoden zur Zellseparation publiziert. Dabei handelt es sich zum einen um eine durch Ultraschall erleichterte Sedimentation (Hawkes et al., 1997) und zum anderen um die dielektrophoretische Separation (Doscoslis et al., 1997). Diese Methoden sind jedoch noch in der Entwicklung. Außerdem sind sie relativ komplex und können vom ökonomischen Standpunkt her kaum mit den bereits beschriebenen konventionellen Methoden der Zellseparation bzw. Zellanreicherung konkurrieren.

Die WO 92/01779 beschreibt einen "scale-up"-fähigen Wannenreaktor zur Durchführung von Fermentationen mit einem länglichen horizontalen Behälter mit schmalem vertikalen Querschnitt mit einer Einlassöffnung und einer Auslassöifnung, die der Länge nach beabstandet sind. Ein Nährstrom fließt von der Auslassöffnung in einen Separator, wie zum Beispiel einen Hydrozyklon oder eine Absetzvorrichtung, der/die die Biomasse, wie beispielsweise Sewage oder Hefe, und andere Festkörper aufkonzentriert und die Flüssigkeit abtrennt.

Die EP-A-0 068 537 bezieht sich auf einen Prozess zur Herstellung von Blutzellprotein und Häm durch Spaltung von Hämoglobin durch Säure und ein organisches Lösungsmittel. Nach weiteren Behandlungsschritten bei Temperaturen von 20° bis 50° findet die Abtrennung der erhaltenen Dispersion in Hydrozyklonen, oder einer Hydrozyklongruppe statt und das zurückgewonnene organische Lösungsmittel wird zumindest teilweise dem Prozess wieder zugeführt.

Es besteht daher ein dringender Bedarf nach einem Verfahren zur Zellanreicherung in Perfusionskulturen sowie generell zur Aberntung von Zellkulturen. Idealerweise sollte die Vorrichtung oder der Apparat, die/der zur Durchführung des Verfahrens verwendet wird, einfach in der Konstruktion sein, geringen Personalbedarf beanspruchen, robust in der Betriebsart und einfach im Hinblick auf eine Maßstabsvergrößerung sein.

Aufgabe der Erfindung ist es, die dem oben geschilderten Stand der Technik anhaftenden Probleme zu beseitigen und den sich daraus ergebenden dringenden Bedarf nach einem alternativen Verfahren zur Abtrennung von lebensfähigen Saüger- oder Insekten-Zellen aus Zellsuspensionen zu befriedigen.

Erfindungsgemäß wird somit gemäß Patentanspruch 1 ein Verfahren zur Abtrennung von lebensfähigen Saüger- oder Insekten-Zellen aus Zellsuspensionen bereitgestellt, bei dem die Zellsuspension über eine Einlaßeinrichtung tangential in den Zylinderraum mindestens eines Hydrozyklons eingeführt und in einer nach unten gerichteten, spiralförmig kreisenden Umlaufbahn durch den Konus des Hydrozyklons strömen gelassen wird, wobei an der sich am unteren Ende des Konus' befindlichen Unterlauf-Auslaßeinrichtung des Hydrozyklons eine mit Zellen angereicherte Suspension und an der zentral in dem Zylinderraum angeordneten und sich in diesen erstreckenden Überlauf-Auslaßeinrichtung eine an Zellen abgereicherte Suspension gesammelt wird und wobei der Druckabfall zwischen der Einlaßeinrichtung und der Überlauf-Auslaßeinrichtung maximal 4 bar, vorzugsweise 2 bar, beträgt,
wobei der Hydrozyklon folgende Konstruktionsmerkmale aufweist:
zylindrischer Abschnitt :
   Länge 2 bis 12 mm
   Durchmesser 5 bis 15 mm
konischer Abschnitt :
   Verjüngungswinkel 5 bis 20°
Einlaßeinrichtung :
   Durchmesser 0,5 bis 4 mm
Überlauf-Auslaßeinrichtung :
   Durchmesser 1 bis 5 mm
   Einführungstiefe 1 bis 6 mm
Unterlauf-Auslaßeinrichtung :
   Durchmesser 0,5 bis 7 mm.

Mit dem erfindungsgemäßen Verfahren läßt sich im Unterlauf des Hydrozyklons schon bei einzelner Anordnung eine Anreicherung um mindestens das 1,2fache der Zellenzahl im Einlauf ohne nennenswerte Beeinträchtigung der Lebensfähigkeit der Zellen erzielen.

Somit eignet sich das erfindungsgemäße Verfahren speziell zum Einsatz mit kontinuierlichen Zellkulturen mit Zellrückführung, wodurch hohe Zelldichten und Produktivitäten für Proteine (Pharmaproteine, Diagnostika, monoklonale Antikörper) erzielt werden können. Außerdem lassen sich dadurch aufwendigere Perfusionsreaktorsysteme, z. B. mit Membrantechnologie, ersetzen, wodurch insgesamt der Betrieb robuster und die Sterilisierbarkeit zuverlässiger wird.

Weitere vorteilhafte und/oder bevorzugte Ausführungsformen der Erfindung sind Gegenstand der Unteransprüche.

In weiterer Ausgestaltung werden somit in dem erfindungsgemäßen Verfahren mehrere gleichartige oder unterschiedliche Hydrozyklone in paralleler oder serieller Anordnung diskontinuierlich oder kontinuierlich betrieben.

Bei den Zellen, die nach dem erfindungsgemäßen Verfahren aus Zellsuspensionen abgetrennt oder angereichert werden können, kann es sich beispielsweise um Säugerzellen, wie Blut- oder Hybridomzellen, oder Insektenzellen handelt.

Von den Figuren zeigt
Figur 1 den prinzipiellen Aufbau eines in dem erfindungsgemäßen Verfahren verwendeten Hydrozyklons,
Figur 2 die Wirkungsweise eines in dem erfindungsgemäßen Verfahren verwendeten Hydrozyklons,
Figur 3 die Kombination eines in dem erfindungsgemäßen Verfahren verwendeten Hydrozyklons mit einem Bioreaktor und
Figur 4 die Kombination zweier in dem erfindungsgemäßen Verfahren verwendeter Hydrozyklone zum seriellen Betrieb.

Im folgenden wird die Erfindung ohne Beschränkung anhand von Ausführungsbeispielen und unter Bezugnahme auf die Figuren detaillierter beschrieben.

Hydrozyklone an sich sind dem Fachmann gut bekannt, und die Prinzipien ihres Betriebes sind detailliert in Hand- und Lehrbüchern beschrieben (Bradley, 1965; Svarovsky, 1984; Heiskanen, 1993). Da ihre Konstruktion und ihr Bau einfach sind, sind ihre Preise im Vergleich zu anderen Geräten, welche die gleiche Aufgabe erfüllen, niedrig. Hinsichtlich ihrer Anwendungen sind Zyklone extrem vielseitig. Sie können benutzt werden, um Suspensionen zu konzentrieren, Flüssigkeiten zu klären, Feststoffe bezüglich Größe und Dichte zu sortieren, unmischbare Flüssigkeiten zu trennen, Flüssigkeiten zu entgasen etc. Es gibt zahlreiche Hydrozyklonhersteller, wie z. B. Dorr-Oliver Inc., Krebs Engs. in den USA, Richard Mozley Ltd in England und AKW, Apparate und Verfahren GmbH in Deutschland, um nur einige wenige zu nennen.

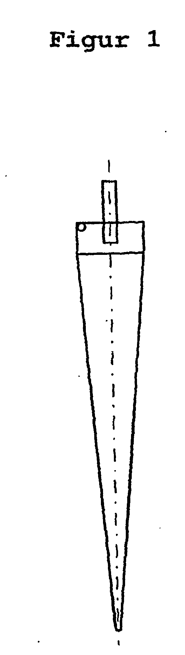

Wie in Figur 1 beispielhaft dargestellt, bestehen Hydrozyklone aus einem konischen Abschnitt mit an sich beliebiger Länge und beliebigem Durchmesser, dem ein kurzer zylinderförmiger Abschnitt folgt, der mit einer Einlaßeinrichtung, z.B. einem entsprechenden Rohr, für die tangentiale Einführung des Trennguts versehen ist. Der Zylinder wird mit einer Platte abgeschlossen, in deren Mitte axial der Überlaufabfluß, z.B. durch eine entsprechende Auslaßeinrichtung, die sich in den Zylinderraum erstreckt, z.B. ein zylindrisches Rohr oder ein Stutzen, erfolgt. Diese zentrale Überlauf-Auslaßeinrichtung wird auch Wirbelsucher oder Tauchrohr genannt. Der Konus endet in einer Auslaßeinrichtung, beispielsweise ein zylindrisches Rohr, über das der Unterlauf abfließt. Diese Unterlauf-Auslaßeinrichtung kann mit einem regelbaren Ventil versehen sein.

Obwohl das erste Patent für Zyklone schon über 100 Jahre alt ist (Bretnei, 1891), kam es zur ersten industriellen Anwendung erst am Ende des 2. Weltkriegs. Das Gerät, das ursprünglich ausgelegt war, um Fest/Flüssig-Trennungen zu erreichen, wird derzeit auch genutzt für Feststoff/Feststoff-(Klima & Kim, 1998), Flüssig/Flüssig- (Moraes et al., 1996) und Gas/Flüssig-Trennungen (Marti et al., 1996).

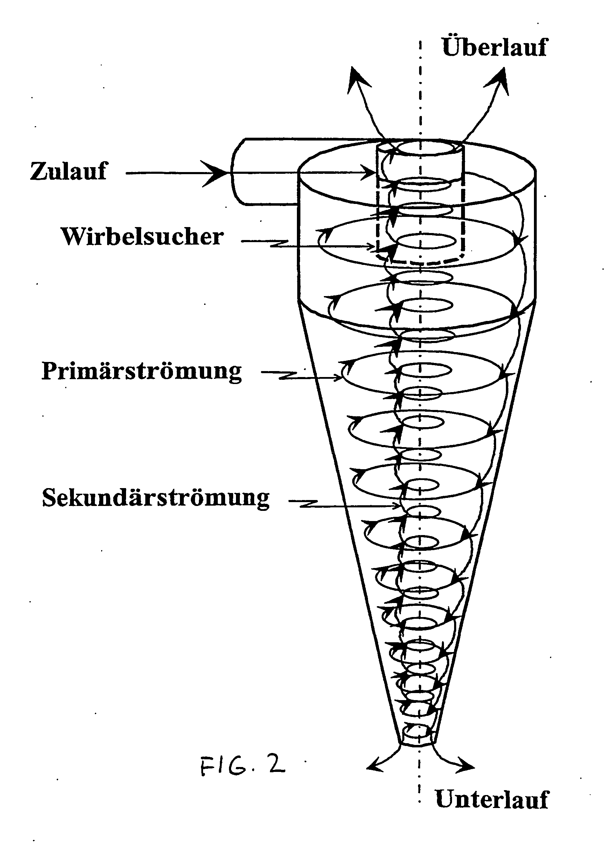

Die Wirkungsweise eines in dem erfindungsgemäßen Verfahren verwendeten Hydrozyklons geht aus Figur 2 hervor. Der Ausdruck "Zulauf" bezieht sich auf eine Zellsuspension, die in den Hydrozyklon durch das Einlaßrohr tangential eintritt. Der Zulauf kann von einem biologischen Reaktor oder einer beliebigen anderen Quelle von Zellen stammen. Der Ausdruck "Unterlauf" bezieht sich auf die mit Zellen angereicherte Suspension, die den Hydrozyklon am Ende der konischen Verjüngung verläßt. Der Ausdruck "Überlauf", bezieht sich auf die an Zellen abgereicherte Suspensionsflüssigkeit, die den Zyklon über das konzentrische Rohr kopfwärts verläßt.

Vorteilhafterweise können in dem erfindungsgemäßen Verfahren zwei oder auch mehrere gleichartige oder unterschiedliche Hydrozyklone in serieller Anordnung betrieben werden, wie z.B. in Kapitel 9 des Buches von Svarovsky (1984) beschrieben ("hydrocyclones in series" bzw. "series of hydrocyclones"). Eine Anordnung von zwei oder mehreren Hydrozyklonen in Serie oder Reihe erreicht man z.B. dadurch, daß der Überlauf des ersten Hydrozyklons zum Einlauf des zweiten wird usw. Natürlich kann auch eine zusätzliche Einrichtung, wie z.B. eine Pumpe, in den Verbindungsleitungen zwischen den beiden betreffenden Hydrozyklonen untergebracht sein.

Auch die parallele Anordnung von zwei oder mehreren Hydrozyklonen ist in dem erfindungsgemäßen Verfahren vorteilhaft. Dieser Parallelbetrieb wird in der Industrie bereits vielfach durchgeführt und ist beispielsweise in Kapitel 5 des o. a. Buches (Svarovsky, 1984) sehr konkret beschrieben ("hydrocyclones in parallel"). Eine Anordnung von zwei parallelen Hydrozyklonen wäre z. B. mit einer Aufteilung des Zulaufs in zwei Teile gegeben, wobei jeder dieser Teile direkt einem Hydrozyklon zugeführt wird.

Im Gegensatz zu Zentrifugen, deren Trennprinzip auf der durch die in den Rotoren auftretende Zentrifugalkraft hervorgerufenen Sedimentation beruht, benötigen Hydrozyklone keine derartigen Rotoren, da die Wirbelbewegung durch die Flüssigkeit selbst induziert wird, wie aus Figur 2 ersichtlich ist.

Durch die tangentiale Einführung des Zulaufs (z.B. an Zellen anzureichernde Zellsuspension) in den oberen Teil des zylindrischen Abschnitts erfährt die Flüssigkeit (z.B. an Zellen anzureichernde Zellsuspension) eine starke, nach unten gerichtete Wirbelbewegung, wodurch auch ein ausgeprägtes Zentrifugalfeld erzeugt wird. Durch dieses Zentrifugalfeld wandern die festen Partikel (z.B. Zellen) radial nach außen in Richtung auf die Wand zu und werden durch die konische Verengung nach unten geführt und schließlich über den Unterlaufauslaß abgelassen. Die Flüssigkeit (z.B. die an Zellen abgereicherte Zellsuspension) dagegen kehrt ihre vertikale Richtung um, bewegt sich mit noch stärkerer Wirbelbewegung nach oben und verläßt den Hydrozyklon durch den Überlaufauslaß. Diese Flüssigkeit enthält nur noch kleinere bzw. leichtere Partikel.

Letztlich bewirkt die Differenz des statischen Drucks zwischen Einlauf- und Auslaufrohr die Wirbelbewegung, die Absetzung im erzeugten Zentrifugalfeld und die Anreicherung der Partikel (z.B. Zellen) im Unterlauf bzw. deren Abreicherung im Überlauf.

Das konventionelle Einsatzgebiet der Hydrozyklone ist die Konzentrierung von Suspensionen, wobei die grobkörnigen Partikel das Gerät in Form einer konzentrierten Suspension als Unterlauf verlassen, während die Feinpartikel, die nicht abgetrennt werden können, in Form einer verdünnten Suspension durch den Überlauf austreten.

In der Vergangenheit wurde die Auffassung vertreten, daß Hydrozyklone nicht effektiv zur Konzentrierung von Suspensionen verwendet werden können, wenn die Dichtedifferenz zwischen den Partikeln und der suspendierenden Flüssigkeit < 0,5 g/cm³ beträgt. Thew (1980) war der erste, der diese Auffassung widerlegte. Er ließ sich einen Hydrozyklon zur Flüssig/Flüssig-Trennung, wie z. B. in Wasser dispergiertes Öl, patentieren, wobei die Dichtedifferenz zwischen dem Wasser und den Öltropfen nur im Bereich von 0,1 - 0,2 g/cm³ lag.

Säügerzellen, Insektenzellen, Pflanzenzellen und Mikroorganismen sind klein, und ihre Dichte liegt nahe bei der des Wassers und beträgt typischerweise etwa 1,05 g/cm³. Aus diesem Grund besteht im Stand der Technik die Auffassung, daß es nicht möglich ist, diese Zellen mit Hilfe von Hydrozyklonen aus wäßrigen Zellsuspensionen abzutrennen. Als Beispiel für diese (irrtümliche). Auffassung sei hier ein schriftlicher Hinweis von Naganna et al. (1996) in deren Patent angeführt: "When a solution containing cells is fed to the liquid cyclone, the cells have a diameter smaller than the critical diameter so that there is no difference in concentration between the upstream and the downstream in the liquid cyclon, which makes separation of the cells impossible".

Wie die nachfolgenden Beispiele zur Veranschaulichung dieser Erfindung zeigen, ist diese Auffassung aber nicht zutreffend. Die Erfindung beruht auf der Erkenntnis, daß Hydrozyklone sehr wohl zur Abtrennung von Zellen aus wäßrigen Zellsuspensionen mit hohem Trennwirkungsgrad benutzt werden können, und zwar ohne Beeinträchtigung der Lebensfähigkeit in praktisch relevantem Ausmaß.

Eine andere, häufig geäußerte Auffassung ist die, daß Säugerzellen eine hohe Empfindlichkeit gegenüber Scherung aufweisen. Erfindungsgemäß wurde aber festgestellt, daß Säugerzellen und ähnliche Zellen Scherungen gut überstehen, insbesondere solche, wie sie in Hydrozyklonen auftreten, solange eine bestimmte kritische Druckdifferenz oder ein bestimmter kritischer Druckabfall (jeweils Manometerdruck) zwischen der Einlaßeinrichtung und der Überlauf-Auslaßeinrichtung im zylindrischen Abschnitt des Hydrozyklons (etwa 4 bar, vorzugsweise 2 bar) nicht überschritten wird. Das hängt zum Teil sicherlich damit zusammen, daß ihre Verweilzeit in dem Hydrozyklon extrem kurz ist.

Nach dem erfindungsgemäßen Verfahren wird eine Zellsuspension, die direkt aus dem Bioreaktor kommt, unter Druck, beispielsweise bis zu 4 bar, vorzugsweise 2 bar, über dem Atmosphärendruck, einem oder mehreren (d.h. parallel angeordneten) Hydrozyklonen zugeführt. Die benutzten Hydrozyklone können von konventioneller Bauart sein. Geeignet sind z. B. die kommerziell gefertigten Hydrozyklone der Firma Dorr-Oliver Inc., wie sie in Kapitel 10 von Bradleys Buch (Bradley, 1965) beschrieben sind.

Vorzugsweise werden erfindungsgemäß zwar im Handel erhältliche Hydrozyklone verwendet, beispielsweise die von den oben genannten Herstellern gefertigten, generell bestehen aber keine besonderen Beschränkungen in bezug auf die Konstruktion oder den Typ. Geeignete Hydrozyklone ermittelt der Fachmann durch einfache Versuche und unter Berücksichtigung der praktischen Gegebenheiten. Entscheidend ist lediglich, daß der beabsichtigte Zweck erreicht wird, nämlich lebensfähige Zellen aus Zellsuspensionen abzutrennen. Geeignet sind beispielsweise Hydrozyklone mit in etwa den folgenden Konstruktionsmerkmalen.

Die Länge des zylindrischen Abschnittes beträgt etwa 2 bis 12 mm und dessen Durchmesser etwa 5 bis 15 mm, vorzugsweise 10 mm. Der Winkel, in dem der konische Abschnitt sich ausgehend vom zylindrischen Abschnitt verjüngt, liegt im Bereich von etwa 5 bis 20°. Dadurch wird gleichzeitig die Länge des konischen Abschnittes festgelegt. Die Einlaßeinrichtung (für die Zellsuspension) in den zylindrischen Teil, beispielsweise ein entsprechendes Rohr, hat zum Beispiel einen Durchmesser im Bereich von etwa 0,5 bis 4 mm, vorzugsweise 2 mm. Die Überlauf-Auslaßeinrichtung, beispielsweise ein entsprechendes Rohr (Tauchrohr), hat zum Beispiel einen Durchmesser von 1 bis 5 mm und erstreckt sich beispielsweise etwa 1 bis 6 mm in der zylindrischen Abschnitt. Die Unterlauf-Auslaßeinrichtung, beispielsweise ein entsprechendes Rohr, hat zum Beispiel einen Durchmesser von 0,5 bis 7 mm.

Der Zulauf (d.h. die an Zellen anzureichernde Zellsuspension) wird tangential in den zylindrischen Teil des Hydrozyklons eingeführt, wodurch eine starke, abwärts gerichtete Wirbelbewegung und dadurch ein Zentrifugalfeld erzeugt wird, auf Grund dessen die Zellen radial nach außen transportiert werden, an die Wand gelangen und den Hydrozyklon als Unterlauf verlassen. Die Unterlaufleitung ist so ausgewählt, bzw. enthält eine Abstelleinrichtung, z.B. ein regelbares Ventil, mit dem der Unterlauf reguliert werden kann, daß ein Teil des Flüssigkeitsstroms seine vertikale Richtung umkehrt und nach oben unter noch stärkerer Wirbelbildung durch die zentrale Überlaufleitung abgeführt wird und dabei kleinere Partikel und Zellbruchstücke mit austrägt. Dagegen sind die Zellen im Unterlauf angereichert.

Das erfindungsgemäße Verfahren zur Abtrennung von Zellen aus Zellsuspensionen ist natürlich nicht nur in Verbindung mit Perfusionszellkulturen und zur Ernte von Zellen einsetzbar, sondern eignet sich generell für beliebige Trennprobleme, für die sich die Verwendung eines Hydrozyklones anbietet. Die verwendeten Hydrozyklone können kontinuierlich, aber auch mit Unterbrechung, d.h. diskontinuierlich, betrieben werden. Die genaue Auswahl der Hydrozyklongröße ist wichtig, um einen hohen Trenneffekt zu erhalten (beispielhafte Konstruktionsmerkmale der einzelnen Hydrozyklonteile sind oben angegeben). Die Lebensfähigkeit der Zellen wird im allgemeinen nicht beeinträchtigt, solange der bereits oben angegebene kritische Druckabfall nicht überschritten wird.

In dem erfindungsgemäßen Verfahren sind auch mehrere Hydrozyklone verwendbar, die seriell oder parallel angeordnet oder geschaltet sein können. Die Serienschaltung kann genutzt werden, um den Trennwirkungsgrad oder Trenneffekt zu erhöhen, d. h. um eine stärkere Konzentrierung an Zellen im Unterlauf zu erreichen.

Die erfindungsgemäße Abtrennung von Zellen aus Zellsuspensionen mit Hilfe von Hydrozyklonen hat im Vergleich zur Abtrennung mit Zentrifugen deutliche Vorteile. Hydrozyklone sind einfach herzustellen und infolgedessen zu niedrigen Preisen erhältlich.

Der Einbau der Hydrozyklone ist einfach und preiswert, da keine besonderen Baumaßnahmen erforderlich sind und nur eine äußerst geringe Installationsfläche benötigt wird. Die Aufrechterhaltung des Betriebszustandes der Hydrozyklone ist einfach, da sie keine bewegten Teile enthalten.

Die Strömungsgeschwindigkeit ist im Vergleich zu ihrer Größe um ein Vielfaches höher und die Verweilzeit der Zellen entsprechend niedrig. Dadurch kann man erwarten, daß durch den Einsatz von Hydrozyklonen zur Aberntung von Zellkulturen sowohl die Investitions- als auch die Betriebskosten gesenkt werden, was sich auch in einem erniedrigten Produktpreis niederschlagen wird.

Diese Vorteile kommen auch dann zum Tragen, wenn Membranfilter und Absetzer zum Vergleich herangezogen werden. Im Vergleich zu Membranfiltern haben Hydrozyklone den zusätzlichen Vorteil, daß sich eine konstante Strömungs- bzw. Flußgeschwindigkeit bzw. -rate für einen gegebenen Druckabfall in einfacher Weise einstellen läßt und ihr Betrieb über Monate, möglicherweise sogar Jahre, ohne Unterbrechung möglich ist.

Die folgenden Beispiele veranschaulichen ohne Beschränkung die Erfindung.

### Beispiel 1

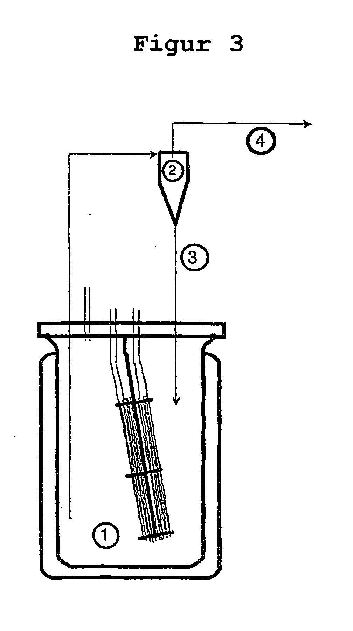

In der in Figur 3 gezeigten Anordnung wurde der Bioreaktor **1** benutzt, um HeLa-Zellen zu kultivieren. 95,4 % der Zellen in der Suspension waren lebensfähig, und die Zellsuspension wurde einem Doxie-Hydrozyklon **2**, hergestellt von Dorr-Oliver Inc., zugeführt. Der gewählte Druckabfall ergab eine Strömungsgeschwindigkeit von 36,7 cm³/s, und es konnten 80 % der Zellen, von denen 95,2 % lebensfähig waren, im Unterlauf **3** abgetrennt werden. Die Zell-Anfangskonzentration betrug 0,43 Vol.-%, die erzielte Zellkonzentration im Unterlauf lag bei 0,7 Vol.-% und die Zellkonzentration im Überlauf **(4)** betrug nur 0,19 Vol.-%, wobei 96,7 % der Zellen lebensfähig waren.

Die Durchführung des gleichen Experiments mit einem Hydrozyklon gleicher Größe wie der des Doxie-Hydrozyklons, aber mit unterschiedlichen geometrischen Abmessungen, hergestellt von Richard Mozley Ltd., ergab eine höhere Flußgeschwindigkeit bzw. -rate, nämlich 50 cm³/s, aber mit geringerer Zellrückhaltung im Unterlauf, nämlich nur 40 %. Das bedeutet, daß nicht nur die Größe des Hydrozyklons wichtig für eine gute Trennung ist, sondern auch seine geometrischen Abmessungen.

### Beispiel 2

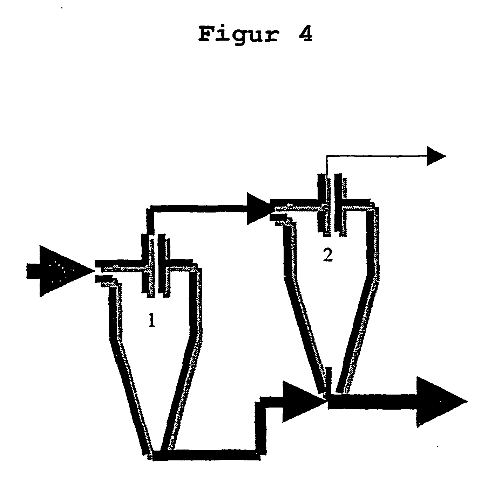

Die Hydrozyklone, beispielsweise Doxie-Hydrozyklone, können auch in serieller Anordnung, wie in Figur 4 dargestellt, verwendet werden. Eine Suspension von HeLa-Zellen, die zu 87,2 % lebensfähig waren, wurde dem ersten Hydrozyklon **1** zugeführt, dessen Überlauf dem zweiten Doxie-Hydrozyklon **2** als Zulauf zugeführt wurde. Bei dem gewählten Druckabfall war es möglich, insgesamt 94 % der Zellen im Unterlauf des ersten und zweiten Hydrozyklons anzureichern, wobei die Überlebensfähigkeit bei 89 % lag.

Zusammengefasst betrifft die Erfindung somit ein neues Verfahren zur Abtrennung von lebensfähigen Zellen aus Zellsuspensionen, z.B. biologischen Kulturmedien, mit Hilfe von Hydrozyklonen. Dabei handelt es sich bei den Zellen bevorzugt um höhere Zellen, d. h. Zellen von Säugern, z.B. Hybridome etc., oder Zellen von Insekten, die durch das erfindungsgemäße Verfahren mit hoher Trenn- bzw. Anreicherungswirkung unter weitgehender Erhaltung der Lebensfähigkeit abgetrennt bzw. angereichert (abgeerntet) werden können. Im Vergleich zu anderen Geräten oder Apparaten, wie spezielle Zentrifugen und Membranfilter im Kreuzstrombetrieb, welche die gleiche Aufgabe erfüllen können, handelt es sich bei den Hydrozyklonen um einfache, billige, zuverlässige und robuste Geräte. Der Ersatz bekannter Abtrenn- und Zellrückhaltesysteme durch Hydrozyklone veringert den Kapitaleinsatz und die Betriebskosten. Insbesondere kann das erfindungsgemäße Verfahren unter Verwendung von Hydrozyklonen auch zur Zellrückhaltung in Perfusionskulturen angewendet werden.

### Literaturstellen

Bradley, D. (1965) The Hydrocyclone. Pergamon Press, Oxford.

Bretnei, E., US Patent No. 453,105 (1891).

Colman, D.A. and Thew, M.T. (1980), Cyclone separator, US Patent No. 4,237,006.

Doscolis, A., Kalogerakis, N., Behrie, L.A. and Kaler, K.V.I.S. (1997), Filter for perfusion cultures of animal cells and the like. US Patent No. 5,626,734.

Hawkes, J.J., Limaye, M.S. and Coakley, W.T. (1997), Filtration of bacteria and Yeast by ultrasound-enhanced sedimentation. Journal of Applied Microbiology, 82 (1), 39-47.

Heiskanen K (1993) Particle Classification. Chapman & Hall, London.

Klima MS & Kim BH (1998) Dense-medium separation of heavymetal particles from soil using a wide-angle hydrocyclone. Journal of Environmental Science and Health, Part A. 33: 1325-1340.

Kroner, K.-H. and Vogel, J. (1998). European Patent No. EP-0 815 927 A2.

Marti S, Erdal FM, Shoham O, Shirazi S & Kouba GE (1996) Analysis of gas carry-under in gas-liquid cylindrical cyclones. In: Claxton D, Svarovsky L & Thew M (eds.) Hydrocyclones' 96 (pp. 399-421) Mechanical Engineering Publications, London & Bury Saint Edmunds.

Moraes CAC, Hackenberg CM, Russo C & Medronho RA (1996) Theoretical analysis of oily water hydrocyclones. In: Claxton D, Svarovsky L & Thew M (eds.) Hydrocyclones'96 (pp. 383-398) Mechanical Engineering Publications, London & Bury Saint Edmunds.

Naganu, Y., Suganuma, T, Satoh, K. and Ikeda, M. (1996), Method for purification of amino acids, nucleic acids using a hydrocyclone. US Patent No. 5,547,858.

Peshwa, M.V. (1999), Mammalian Cell Culture. In: Demain, A.L. and Davies, J.E. (eds.) Manual of Industrial Microbiology and Biotechnology, 2^{nd} Ed., ASM Press, Washington.

Svarovsky L (1984) Hydrocyclones. Holt, Rinehart and Winston, London.

Tokashiki, M. and Yokoyama, S. (1997), Bioreactors designed for animal cells. In: Hauser, H. and Wagner, R. (eds.), Mammalian Cell Biotechnology in Protein Production, Walter de Gruyter, Berlin.

Yhland, C. (1992), Centrifugal Separator. US Patent No. 5, 160, 310.

Zeng, A.-P. and Deckwer, W.-D. (1999), Model Simulation and analysis of perfusion culture of mammalian cells at high cell density. Biotechnology Progress, 15, 373-382.

## Patentansprüche

1. Verfahren zur Abtrennung von lebensfähigen Säuger- oder Insekten-Zellen aus Zellsuspensionen, bei dem die Zellsuspension über eine Einlaßeinrichtung tangential in den Zylinderraum mindestens eines Hydrozyklons eingeführt und in einer nach unten gerichteten, spiralförmig kreisenden Umlaufbahn durch den Konus des Hydrozyklons strömen gelassen wird, wobei an der sich am unteren Ende des Konus' befindlichen Unterlauf-Auslaßeinrichtung des Hydrozyklons eine mit Zellen angereicherte Suspension und an der zentral in dem Zylinderraum angeordneten und sich in diesen erstreckenden Überlauf-Auslaßeinrichtung eine an Zellen abgereicherte Suspension gesammelt wird und wobei der Druckabfall zwischen der Einlaßeinrichtung und der Überlauf-Auslaßeinrichtung maximal 4 bar beträgt, wobei der Hydrozyklon folgende Konstruktionsmerkmale aufweist:
zylindrischer Abschnitt:
Lange 2 bis 12 mm
Durchmesser 5 bis 15 mm
konischer Abschnitt:
Verjüngungswinkel 5 bis 20°
Einlaßeinrichtung:
Durchmesser 0,5 bis 4 mm
Überlauf-Auslaßeinrichtung:
Durchmesser 1 bis 5 mm
Einführungstiefe 1 bis 6 mm
Unterlauf-Auslaßeinrichtung:
Durchmesser 0,5 bis 7 mm.

2. Verfahren nach Anspruch 1, wobei der Druckabfall zwischen der Einlaßeinrichtung und der Überlauf-Auslaßeinrichtung 2 bar beträgt.

3. Verfahren nach Anspruch 2, wobei der Hydrozyklon folgende Konstruktionsmerkmale aufweist:
zylindrischer Abschnitt:
Länge 2 bis 12 mm
Durchmesser 10 mm
konischer Abschnitt:
Verjüngungswinkel 5 bis 20°
Einlaßeinrichtung:
Durchmesser 2 mm
Überlauf-Auslaßeinrichtung:
Durchmesser 1 bis 5 mm
Einführungstiefe 1 bis 6 mm
Unterlauf-Auslaßeinrichtung:
Durchmesser 0,5 bis 7 mm.

4. Verfahren nach einem der vorherigen Ansprüche, wobei mehrere gleichartige oder unterschiedliche Hydrozyklone in paralleler oder serieller Anordnung diskontinuierlich oder kontinuierlich betrieben werden.

5. Verfahren nach einem der vorhergehenden Ansprüche, wobei es sich bei den Säugerzellen um Blut- oder Hybridomzellen handelt.

## Claims

1. Method of separating viable mammalian or insect cells from cell suspensions, wherein the cell suspension is introduced, by way of an inlet device, tangentially into the cylinder space of at least one hydrocyclone and is allowed to flow in a downwardly directed, spirally cycling course through the cone of the hydrocyclone, a suspension of increased cell concentration being collected at the bottom run-off outlet device of the hydrocyclone, located at the bottom end of the cone, and a suspension of decreased cell concentration being collected at the top run-off outlet device, which is arranged centrally in, and extends into, the cylinder space, and the pressure drop between the inlet device and the top run-off outlet device being at most 4 bar, the hydrocyclone having the following features of construction:
cylindrical portion:
length 2 to 12 mm
diameter 5 to 15 mm
conical portion:
angle of taper 5 to 20°
inlet device:
diameter 0.5 to 4 mm
top run-off outlet device:
diameter 1 to 5 mm
depth of introduction 1 to 6 mm
bottom run-off outlet device:
diameter 0.5 to 7 mm.

2. Method according to claim 1, wherein the pressure drop between the inlet device and the top run-off outlet device is 2 bar.

3. Method according to claim 2, wherein the hydrocyclone has the following features of construction:
cylindrical portion:
length 2 to 12 mm
diameter 10 mm
conical portion:
angle of taper 5 to 20°
inlet device:
diameter 2 mm
top run-off outlet device:
diameter 1 to 5 mm
depth of introduction 1 to 6 mm
bottom run-off outlet device:
diameter 0.5 to 7 mm.

4. Method according to one of the preceding claims, wherein a plurality of identical or different hydrocyclones arranged in parallel or in series are operated discontinuously or continuously.

5. Method according to one of the preceding claims, wherein the mammalian cells are blood cells or hybridoma cells.

## Revendications

1. Procédé pour séparer des cellules viables de mammifère ou d'insecte de suspensions cellulaires, la suspension cellulaire étant introduite de façon tangentielle par le biais d'un dispositif d'admission dans l'espace du cylindre au moins d'un hydrocyclone et en mesure de s'écouler selon une trajectoire circulaire de forme hélicoïdale dirigée vers le bas par le cône de l'hydrocyclone, dans lequel une suspension enrichie avec des cellules est collectée au niveau du dispositif de sortie de la conduite inférieure se trouvant au niveau de l'extrémité inférieure du cône de l'hydrocyclone et au niveau du dispositif de sortie de la conduite supérieure agencé de façon centrale dans l'espace du cylindre et s'étendant dans ce dernier, et dans lequel la chute de pression entre le dispositif d'admission et le dispositif de sortie de la conduite supérieure s'élève à 4 bars maximum, l'hydrocyclone présentant les caractéristiques de construction suivantes :
Section cylindrique :
Longueur 2 à 12 mm
Diamètre 5 à 15 mm
Section conique :
Angle du col 5 à 20°
Dispositif d'entrée :
Diamètre 0,5 à 4 mm
Dispositif de sortie de la conduite supérieure :
Diamètre 1 à 5 mm
Profondeur d'introduction 1 à 6 mm
Dispositif de sortie de la conduite inférieure:
Diamètre 0,5 à 7 mm

2. Procédé selon la revendication 1, dans lequel la chute de pression entre le dispositif d'admission et le dispositif de sortie de la conduite supérieure s'élève à 2 bars.

3. Procédé selon la revendication 2, dans lequel l'hydrocyclone présente les caractéristiques de construction suivantes :
Section cylindrique :
Longueur 2 à 12 mm
Diamètre 10 mm
Section conique :
Angle du col 5 à 20°
Dispositif d'entrée :
Diamètre 2 mm
Dispositif de sortie de la conduite supérieure :
Diamètre 1 à 5 mm
Profondeur d'introduction 1 à 6 mm
Dispositif de sortie de la conduite inférieure :
Diamètre 0,5 à 7 mm

4. Procédé selon l'une quelconque des revendications précédentes, dans lequel plusieurs hydrocyclones différents ou de même type sont actionnés dans un agencement en parallèle ou en série de façon continue ou discontinue.

5. Procédé selon l'une quelconque des revendications précédentes, dans lequel il s'agit pour les cellules de mammifères de cellules d'hybridomes et de cellules sanguines.
